# EUROPÄISCHE PATENTANMELDUNG

(11) **EP 1 702 678 A1**
(43) Veröffentlichungstag der Anmeldung: **20.09.2006**
(21) Anmeldenummer: 06005354.3
(22) Anmeldetag: 16.03.2006
(51) Int. Cl.: B01J 19/08, B01J 19/12, B01J 19/18, B01J 10/02

(54) **Einrichtung zur Behandlung einer Flüssigkeit mit einer energetischen Strahlung**

(30) Priorität: 16.03.2005 DE 102005012053
(71) Anmelder: Glatt Systemtechnik GmbH, 01277 Dresden (DE)
(72) Erfinder: Kretzschmar, Ralf, 01309 Dresden (DE); Pritzke, Heinz, 01737 Kesselsdorf OT Braunsdorf (DE); Paulusch, Uwe, 01309 Dresden (DE); Kempe, Wolfgang, 01169 Dresden (DE)
(74) Vertreter: Pätzelt, Peter

(57) **Zusammenfassung**

Die Erfindung betrifft eine Einrichtung zur Behandlung einer Flüssigkeit mit einer energetischen Strahlung. Die Einrichtung besteht aus einem um eine vertikale Achse (6) drehbaren Flüssigkeitsträger (2) mit einer Ablauffläche für die zu handelnde Flüssigkeit, die mindestens teilweise die Form eines Kegels oder Kugelsegmentes aufweist, einem oberen Flüssigkeitsauslass (16) im Bereich der vertikalen Achse (6) und mindestens einer Strahlungsquelle, die derart angeordnet ist, dass die energetische Strahlung von außen auf die Flüssigkeit auf der Ablauffläche des Flüssigkeitsträgers (2) einwirkt.

Die Einrichtung kann innerhalb einer Behandlungskammer sein, in der ein Überdruck, Normaldruck oder ein Vakuum erzeugt wird. Die Strahlungsquelle kann eine Einrichtung zur Erzeugung einer lonen-, Elektronen-, Plasma- sowie Lichtstrahlung sein oder ein elektrisches oder magnetischen Feld. Desweiteren können UV- oder Infrarot-Strahlquellen oder Dampfquellen, wie Magnetron-Zerstäubungseinrichtungen, oder eine Vakuum-Verdampfungseinrichtung eingesetzt werden.

## Beschreibung

Die Erfindung betrifft eine Einrichtung zur Behandlung einer Flüssigkeit mit einer energetischen Strahlung, bei der die zu behandelnde Flüssigkeit von oben auf eine zur Vertikalen geneigten Ablauffläche eines Flüssigkeitsträgers aufgebracht wird und die energetische Strahlung von einer Strahlungsquelle auf die nach unten fließende Flüssigkeit einwirkt.

Nach dem Stand der Technik sind verschiedene Einrichtungen bekannt, bei denen insbesondere eine UV-Strahlung auf die Flüssigkeit einwirkt. Beispielhaft offenbart die DE 43 17 939 C2 eine Vorrichtung zur Behandlung flüssiger Abfälle, insbesondere Abwasser. Die Vorrichtung weist einen Bestrahlungsraum mit gegenüberliegenden, senkrecht stehenden oder gegenüber der Vertikalen geneigten Wänden auf. Am oberen Ende der Wände sind Anlaufprofile vorgesehen, über die die Flüssigkeit zugeführt wird. Zwischen den Wänden ist eine Bestrahlungseinheit mit horizontalen oder vertikalen UV-Strahlungsquellen und Reflektoren vorgesehen.

Die DE 198 31 768 A1 gibt eine Vorrichtung zur Bestrahlung, insbesondere zur UV-Bestrahlung, einer Flüssigkeit an. Die Vorrichtung eignet sich zur Inaktivierung von Viren und anderen pathogenen Erregern in biologischen Flüssigkeiten, z.B. in Blutseren. Die Vorrichtung weist einen rotierenden Zylinder mit einem oberen Zufluss, einem unteren Abfluss und einer Bestrahlungseinheit auf, wobei der Zylinder einen Neigungswinkel mit der Horizontalen einschließt.

In der modernen Verfahrenstechnik werden zunehmend Verfahren und Einrichtungen zur Beeinflussung oder Änderung von Eigenschaften eines Stoffes benötigt, mit denen der Stoff als solcher oder dessen Oberfläche gezielt und wirksam verändert werden kann. Vielfach ist dabei der Stoff ein festes Material, z.B. folienartige Kunststoffe, Glas oder Gewebe. Zunehmend sollen aber auch Flüssigkeiten jeder Art, z.B. Abwässer, Öle oder sonstige chemische sowie pharmazeutische Flüssigkeiten, mit unterschiedlichen energetischen Strahlen wirksam und gleichmäßig behandelt werden.

Der Erfindung liegt damit als Aufgabe zugrunde, eine Einrichtung zur technisch einfachen und wirtschaftlichen Behandlung einer Flüssigkeit mit einer energetischen Strahlung anzugeben. Die Einrichtung soll einen Fiüssigkeitsträger mit einer zur Vertikalen geneigten Ablauffläche, einen oberen Flüssigkeitsauslass und mindestens eine Strahlungsquelle umfassen. Mit der Einrichtung soll die Behandlung der Flüssigkeit wirkungsvoll und gleichmäßig mit unterschiedlichen Stahlquellen sowie in unterschiedlicher Atmosphäre möglich sein.

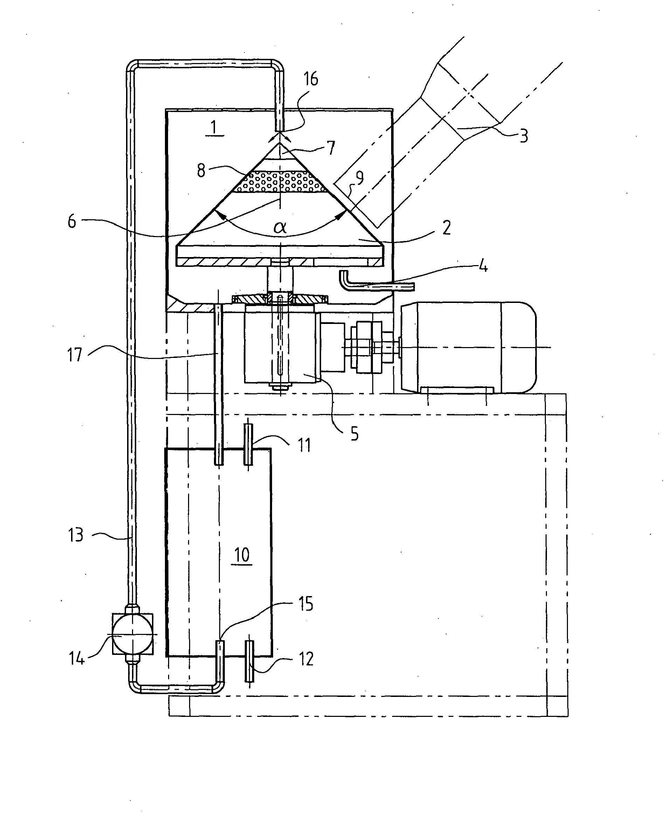

Die Erfindung löst die Aufgabe durch die Merkmale des Anspruchs 1. Vorteilhafte Weiterbildungen der Erfindung sind in den Unteransprüchen gekennzeichnet und werden nachstehend zusammen mit der Beschreibung der Ausführungsbeispiele zur Erfindung, einschließlich der Zeichnung, näher dargestellt.

Der Kern der Erfindung besteht darin, dass die Ablauffläche des Flüssigkeitsträgers (2) mindestens teilweise die Form eines Kegels oder Kugelsegmentes aufweist, der um seine vertikale Achse drehbar ist. Der obere Flüssigkeitsauslass ist im Bereich der vertikalen Achse des Flüssigkeitsträger (2) vorgesehen und mindestens eine Strahlungsquelle ist derart angeordnet, dass die energetische Strahlung von außen auf die Flüssigkeit auf der Ablauffläche des Flüssigkeitsträger (2) einwirkt.

Als energetische Strahlungsquelle können alle Quellen eingesetzt werden, die eine energetische Strahlung oder ein energetisches Feld erzeugen, die geeignet ist, innerhalb einer Flüssigkeit zu chemischen oder physikalischen Reaktionen zu führen, mit denen die Eigenschaften der Flüssigkeit verändert werden. Dazu gehören Ionen-, Elektronen-, Plasma- und Lichtstrahlung sowie elektrische und magnetische Felder. Die Strahlung kann auch spezifische Teilchen, z.B. aus einer Dampfquelle wie Magnetron-Zerstäubungseinrichtungen oder Vakuumverdampfer, beinhalten. Die Behandlung kann auch in einer inerten oder reaktiven Atmosphäre durchgeführt werden. Dazu ist die Einrichtung in einer Behandlungskammer anzuordnen, in der Überdruck, Normaldruck oder ein Vakuum ausgebildet werden kann.

Die Ausbringung der Flüssigkeit aus dem Flüssigkeitsauslass erfolgt im Bereich der vertikalen Achse und kann frei fließend, unter Druck oder Fliehkraft erfolgen, derart dass auf der Ablauffläche des Flüssigkeitsträgers möglichst ein gleichmäßiger Flüssigkeitsfilm ausgebildet wird. Der Auslass kann direkt in der vertikalen Achse vorgesehen sein oder auch seitlich davon.

Die Zuführungsleitung für die Flüssigkeit kann sowohl von unten im Inneren des Flüssigkeitsträgers oder frei von oben zugeführt werden.

Die Oberfläche der Ablauffläche kann mechanische Fließbarrieren aufweisen, damit die Abflussgeschwindigkeit der Flüssigkeit verringert und damit die Verweildauer im Bereich der energetischen Strahlen verlängert wird. Die Fließbarrieren können in beliebiger Form ausgebildet sein. Vorteilhaft sind einfache Aufrauungen der Oberfläche oder Oberflächenprägungen, z.B. Noppen o.ä.

Die Fließbarrieren können auch mit der Form des Flüssigkeitsträgers im Bereich der energetischen Strahlung kombiniert werden, indem dieser Bereich z.B. glockenartig geformt ist und der Wirkbereich der energetischen Strahlung im konkaven Bereich liegt.

Der Flüssigkeitsträger kann im Bereich der Ablauffläche auch Gasdurchlässe aufweisen. Durch die Gasdurchlässe können sehr wirksam vom Inneren des Flüssigkeitsträgers reaktive Gase, z.B. Sauerstoff, durch die abfließende Flüssigkeit geleitet werden.

Eine oder mehrere Strahlungsquellen können, vorzugsweise demontierbar, in der Wandung einer Behandlungskammer angeordnet sein. In einer weiteren Ausführung kann die Strahlungsquelle, z.B. eine Elektronenstrahlquelle, und der Flüssigkeitsträger alternativ mit den Ausgangsklemmen einer Spannungsquelle verbunden sein. Damit kann während der Bestrahlung eine Spannungsdifferenz eingestellt werden.

Die Erfindung wird nachstehend an drei Ausführungsbeispielen näher erläutert. Zugehörig zum Ausführungsbeispiel I zeigt die Zeichnung die Seitenansicht einer erfindungsgemäßen Einrichtung.

### Ausführungsbeispiel I

Die Zeichnung zeigt einen Schnitt durch eine erfindungsgemäße Einrichtung zur Behandlung einer Flüssigkeit in einer abgeschlossenen Behandlungskammer 1 mit einem Flüssigkeitsträger 2, einer Elektronenstrahlquelle 3, die von außen die Wand der Behandlungskammer 1 durchdringt, sowie ein Umwälzsystem für die Flüssigkeit.

Die Behandlungskammer 1 ist beispielhaft vakuumdicht ausgeführt, wobei die Einrichtung zur Erzeugung eines Vakuums in der Zeichnung nicht dargestellt ist. Weiterhin weist die Behandlungskammer 1 einen Gaseinlass 4 auf, durch den ein technologisch erforderliches Inert- oder Reaktivgas in den Bereich unterhalb des Flüssigkeitsträgers 2 in die Behandlungskammer 1 eingelassen werden kann. Ein Gasauslass ist in der Zeichnung nicht dargestellt.

Der Flüssigkeitsträger 2 wird von einem elektromotorischen Antrieb 5 angetrieben und rotiert um eine vertikale Achse 6. Der Flüssigkeitsträger 2 hat die Form eines Kegelmantels mit einem Kegelwinkel α und vertikal oben liegender Kegelspitze 7. Im oberen Bereich des Kegelmantels ist ein Abschnitt mit einer Lochung 8 vorgesehen. Die einzelnen Löcher weisen nach außen aufgebördelte Ränder auf, die als Gasdurchlässe genutzt werden können und für eine abfließende Flüssigkeit als Fließbarrieren wirken.

Die flächige Elektronenstrahlaustrittsöffnung 9 der Elektronenstrahlquelle 3 ist mit geringem Abstand zum Kegelmantel des Flüssigkeitsträgers 2 angeordnet, so dass sie im wesentlichen parallel zum Kegelmantel ausgerichtet ist. Dadurch kann der Elektronenstrahl mit maximaler Energie auf eine auf dem Kegelmantel des Flüssigkeitsträgers 2 abfließende Flüssigkeit einwirken. Die Einrichtung kann bei Bedarf auch mit weiteren ringförmig angeordneten Elektronenstrahlquellen 3 aufgerüstet werden.

Das Umwälzsystem für die Flüssigkeit besteht aus einem Flüssigkeitsbehälter 10 mit einem Zulauf 11 und einem Abfluss 12 sowie einer Umwälzleitung 13, in die eine Umwälzpumpe 14 eingeschaltet ist. Die Umwälzleitung 13 führt von einer Saugöffnung 15 im unteren Bereich des Flüssigkeitsbehälters 10 zu einem Flüssigkeitsuslass 16 oberhalb der Kegelspitze 7 und innerhalb der Behandlungskammer 1. Weiterhin umfasst das Umwälzsystem eine Rückführleitung 17 vom Boden der Behandlungskammer 1 zum Flüssigkeitsbehälter 10.

Der Einsatz der konkreten Einrichtung ist spezifisch von der technologischen Aufgabe abhängig. So kann die vorstehend beschriebene Einrichtung in einfacher Weise auch modifiziert werden, indem die Elektronenstrahlquelle 3 gegen andere Einrichtungen zur Erzeugung energetischer Strahlung ausgetauscht wird. Derartige andere Einrichtungen können beispielhaft UV- oder Infrarot-Strahlquellen, Plasmaquellen, Magnetfelderzeugungseinrichtungen oder auch Dampfquellen wie eine Magnetron-Zerstäubungseinrichtung oder eine Vakuum-Verdampfungseinrichtung sein.

Nachfolgend soll die erfindungsgemäße Einrichtung im Betrieb näher beschrieben werden. Beispielhaft soll die vorstehend beschriebene Einrichtung zur Aktivierung einer zähen Flüssigkeit eingesetzt werden. Die Flüssigkeit ist eine Mischung aus einer Trägerflüssigkeit und verschiedenen Reagenzien. Unter dem Einfluss einer reaktiven Atmosphäre bei einer Temperatur von über 100 °C, die nur kurzzeitig erforderlich ist, können die Eigenschaften der Flüssigkeit spezifisch verändert werden.

Die Flüssigkeit wird in den Flüssigkeitsbehälter 10 eingefüllt. Der Flüssigkeitsträger 2 wird über den Antrieb 5 in Rotation versetzt, über den Gaseinlass 4 wird ein Reaktivgas in die Behandlungskammer 1 eingelassen und über einen kontrollierten Gasauslass wird ein technologisch erforderlicher Partialdruck von 1000 mbar eingestellt. Weiterhin wird die Elektronenstrahlquelle 3 in Betrieb genommen, wobei in der Startphase eine geringere Leistung eingestellt werden kann als in der nachfolgenden konkreten Phase zur Behandlung der Flüssigkeit.

Nach der Einstellung der erforderlichen Verfahrensparameter wird die Flüssigkeit aus dem Flüssigkeitsbehälter 10 mittels der Umwälzpumpe 14 durch die Umwälzleitung 13 zum Flüssigkeitsauslass 16 und auf die Kegelspitze 7 befördert. Unter der Wirkung der vom Flüssigkeitsträger 2 tangential ausgehenden Fliehkraft und der Schwerkraft läuft die Flüssigkeit in Abhängigkeit ihrer Viskosität langsam spiralförmig auf der äußeren Oberfläche des Flüssigkeitsträgers 2 nach unten. Dabei wird die Flüssigkeit sehr gleichmäßig über den gesamten Kegelmantel des Flüssigkeitsträger 2 verteilt.

Die Flüssigkeit kommt während der Bewegung insbesondere im Bereich der Lochung 8 mit dem Reaktivgas in Kontakt, welches vom Gaseinlass 4 in den Innenraum des Flüssigkeitsträgers 2 und durch die Öffnungen in der Lochung 8 strömt. Durch die nach außen aufgebördelten Ränder der einzelnen Löcher der Lochung 8 wird verhindert, dass die Flüssigkeit in das Innere des Flüssigkeitsträger 2 eindringt und weiterhin wird die Fließgeschwindigkeit der Flüssigkeit verringert.

Im weiteren Verlauf fließt die Flüssigkeit spiralförmig nach unten und bildet einen außerordentlich gleichmäßigen Flüssigkeitsfilm aus. Wegen des zunehmenden Umfanges des Kegelmantels des Flüssigkeitsträgers 2 wird die Filmdicke der Flüssigkeit bis in den Bereich der Elektronenstrahlaustrittsöffnung 9 stark verringert, wodurch die Flüssigkeit im Bereich des Elektronenstrahls besonders wirksam behandelt werden kann.

Vom unteren Ende des Flüssigkeitsträgers 2 läuft die Flüssigkeit am Boden der Behandlungskammer 1 durch die Rückführleitung 17 in den Flüssigkeitsbehälter 10 zurück. In Abhängigkeit der technologischen Vorgaben durchläuft die Flüssigkeit eine Zeit lang wiederholt diesen Kreislauf, bis der Prozess beendet und die behandelte und aktivierte Flüssigkeit durch den Auslass 12 aus dem Flüssigkeitsbehälter 10 entfernt werden kann.

In den Fällen, bei denen die vorgesehene Behandlung der Flüssigkeit bereits mit einem einmaligen Durchfluss der Flüssigkeit durch den Wirkbereich des Elektronenstrahls ausreichend ist, kann der Flüssigkeitsbehälter 10 entfallen und die Anlage als Durchlaufanlage betrieben werden.

In Abhängigkeit der technologischen Aufgabe können auch mehrere gleiche oder unterschiedlichen Einrichtungen zur Erzeugung einer energetischen Strahlung in einer Einrichtung oder kaskadenartig in einer oder mehreren Einrichtungen angeordnet sein.

### Ausführungsbeispiel II

In einem Ausführungsbeispiel II soll eine Flüssigkeit mit UV-Licht (ultraviolettes Licht) behandelt werden. Dazu ist die Elektronenstrahlquelle 3 nach dem Ausführungsbeispiel I gegen eine oder mehrere UV-Quelle/-n ausgetauscht worden. Es ist aber auch möglich, dass die UV-Quelle zusätzlich in oder an der Behandlungskammer 1 angeordnet ist. Beispielhaft ist kein Reaktivgas erforderlich, so dass der Gaseinlass 4 außer Betrieb ist. Im übrigen wird die Einrichtung in äquivalenter Weise wie im Ausführungsbeispiel I betrieben.

### Ausführungsbeispiel III

In einem Ausführungsbeispiel III soll die Einrichtung nach Ausführungsbeispiel I zur Behandlung einer Flüssigkeit mit Ionenstrahlen eingesetzt werden. Dazu wird die Elektronenstrahlquelle 3 gegen eine Ionenstrahlquelle ausgetauscht. Verfahrensgemäß wird in der Behandlungskammer 1 und damit auch im Flüssigkeitsbehälter 10 ein Vakuum mit einem Druck von 500 mbar erzeugt.

Die zu behandelnde Flüssigkeit wird wie im Ausführungsbeispiel I in Umwälzung versetzt und fließt auf der oberen Oberfläche des Flüssigkeitsträgers 2 nach unten. Der Flüssigkeitsträger 2, liegt wie die Behandlungskammer 1 an Masse, wogegen der Ionenstrahl ein negatives Potential aufweist. Dadurch werden die negativen Ionen verstärkt und gezielt auf die Oberfläche des Flüssigkeitsträgers 2 geschossen und damit durch sehr wirksam den darauf befindlichen Flüssigkeitsfilm.

## Patentansprüche

1. Einrichtung zur Behandlung einer Flüssigkeit mit einer energetischen Strahlung, bestehend aus einem Flüssigkeitsträger mit einer zur Vertikalen geneigten Ablauffläche, einem oberen Flüssigkeitsauslass und mindestens einer Strahlungsquelle, von der aus die energetische Strahlung auf eine auf der Ablauffläche des Flüssigkeitsträgers befindliche Flüssigkeit einwirkt, **dadurch gekennzeichnet, dass**
- die Ablauffläche des Flüssigkeitsträgers (2) mindestens teilweise die Form eines Kegels oder Kugelsegmentes aufweist,
- dass der Flüssigkeitsträger (2) um seine vertikale Achse (6) drehbar ist,
- dass der obere Flüssigkeitsauslass (16) im Bereich der vertikalen Achse (6) des Flüssigkeitsträgers (2) vorgesehen ist
- und dass mindestens eine Strahlungsquelle derart angeordnet ist, dass die energetische Strahlung von außen auf eine Flüssigkeit auf der Ablauffläche des Flüssigkeitsträgers (2) einwirkt.

2. Einrichtung nach Anspruch 1, **dadurch gekennzeichnet, dass** der obere Flüssigkeitsauslass (16) an einer Leitung vorgesehen ist, die innerhalb oder außerhalb des Flüssigkeitsträgers (2) angeordnet ist.

3. Einrichtung nach Anspruch 1 oder 2, **dadurch gekennzeichnet, dass** die Einrichtung innerhalb einer Behandlungskammer (1) angeordnet ist, in der ein Überdruck, Normaldruck oder ein Vakuum erzeugt werden kann.

4. Einrichtung nach einem der Ansprüche 1 bis 3, **dadurch gekennzeichnet, dass** der Flüssigkeitsträger (2) im Bereich der Ablauffläche Gasdurchlässe und/oder mechanische Fließbarrieren aufweist.

5. Einrichtung nach einem der Ansprüche 1 bis 4, **dadurch gekennzeichnet, dass** am oberen Flüssigkeitsauslass (16) ein Druck- oder Fliehkraftverteiler vorhanden ist.

6. Einrichtung nach einem der Ansprüche 1 bis 5, **dadurch gekennzeichnet, dass** die Strahlungsquelle eine Einrichtung zur Erzeugung einer Ionen-, Elektronen-, Plasma- sowie Lichtstrahlung oder eines elektrischen oder magetischen Feldes ist.

7. Einrichtung nach einem der Ansprüche 1 bis 6, **dadurch gekennzeichnet, dass** der Flüssigkeitsträger (2) und die Strahlungsquelle alternativ mit den Ausgangsklemmen einer Spannungsquelle verbunden werden können.
